# EUROPÄISCHE PATENTANMELDUNG

(11) **EP 1 445 012 A1**
(43) Veröffentlichungstag der Anmeldung: **11.08.2004**
(21) Anmeldenummer: 04001879.8
(22) Anmeldetag: 29.01.2004
(51) Int. Cl.: B01D 61/14, B01D 69/12, A61M 1/36

(54) **Filtervorrichtung**

(30) Priorität: 04.02.2003 DE 10304365
(71) Anmelder: LS medcap GmbH, 72379 Hechingen (DE)
(72) Erfinder: Bell, Carl-Martin Dr., 72762 Reulingen (DE)
(74) Vertreter: Möbus, Daniela, Dr.-Ing.

(57) **Zusammenfassung**

Filtervorrichtung zum Ausfiltern von Leukozyten aus Blut, Blutplasma, Blutkomponenten oder Proteinlösungen, die ein Vlies und mindestens eine Membran mit einer Dicke von kleiner gleich 150 µm und einer Porengröße von kleiner als 15 µm, aufweist.

## Beschreibung

Die Erfindung betrifft eine Filtervorrichtung zum Ausfiltern von Leukozyten aus Blut, Blutplasma, Blutkomponenten oder Proteinlösungen.

In den vergangenen Jahren wurden überall in Europa Gesetze erlassen, dass Blut und Blutkomponenten für Transfusionen, soweit von Leukozyten befreit werden müssen, dass höchstens noch 10⁶ Leukozyten pro Einheit Blutkomponente vorhanden sind. Durch diese Maßnahme können schädliche Nebenwirkungen bei Bluttransfusionen, wie Veränderung des Immunsystems, allergische Sensibilisierung, Virusinfektionen usw. deutlich reduziert werden. Dennoch besteht immer noch ein Krankheitsrisiko bei Transfusionen von 1:500 und ein Sterblichkeitsrisiko von 1:200.000.

Zurzeit bestehen die Hauptprobleme bei den Blutbehandlungsverfahren in der nur ungenügenden Wiedergewinnung von Blutplättchen für die Herstellung von Blutplättchenkonzentraten und in der Aktivierung von Blutkomponenten, d. h. von Zellen, die zu Infektionsreaktionen oder anderen zytokinbedingten Reaktionen des Patienten führen, was für den Heilungsprozess des Patienten schädlich ist.

V. Kratschmar stellte auf dem deutschen Anästhesistenkongress, 22. - 25. Juni 2002 in Nürnberg, folgende Reaktionen die mit nicht hämolytischen Transfusionen zusammenhängen, fest:
1. Leukozyten-Antikörper im Patienten,
2. allergische Reaktionen des Patienten aufgrund von allergenen Antikörpern im Spenderblut,
3. Thrombozyten-Antikörper im Patienten,
4. Vorhandensein von Zytokinen, die zur Zellaktivierung und zu Reaktionen des Patienten wie bei Infektionen führen.

Typ 1 der Reaktionen ging seit der Einführung der Leukozytenfilterung von Blutkomponenten stark zurück.

Die Reaktionen nach Typ 2 nahmen ebenfalls dadurch ab, dass man zunehmend Spender mit Allergien vom Blutspenden ausschloss.

Die Reaktionen des Typs 3 sind auf einem konstant niedrigen Niveau geblieben, während die Reaktionen vom Typ 4 stark angestiegen sind, seit die Leukozytenfilterung von Blut und seinen Komponenten eingeführt wurde. Kratschmar führte das Vorhandensein von Zytokinen und Zellaktivierungssignalen auf die Aktivierung von Spenderblutzellen während der Leukozytenfilterung durch eine Interaktion der Blutbestandteile mit den Fremdmaterialien der Filtermedien zurück. Voraktivierte, noch vorhandene Leukozyten verursachen eine solche Aktivierung vor allem in Blutplättchenkonzentraten während deren Lagerung, die normalerweise bis zu 5 Tagen bei 22 °C beträgt. Eine Aktivierung von Thrombozyten durch Filtermedien ist ein schneller Prozess, der zu einem Anstieg der Neigung der Blutplättchen zum Aneinanderhaften und zu Blutgerinnungen führt.

Auf diese Weise kann zu Infektionsreaktionen und/oder Gerinnungsaktivitäten aktiviertes Blut in den Patienten gelangen und dann den Heilungsprozess des Patienten negativ beeinflussen.

Ein weiterer Nachteil bei bisherigen Leukozytenfiltervorrichtungen besteht darin, dass die Filtervorrichtungen bis zu 10 % des Spenderbluts aufsaugen, was eine Verschwendung darstellt.

Leukozytenfilterungen werden bisher mit fasrigen oder porösen Materialien, beispielsweise Vliesen durchgeführt, die jedoch relativ voluminös sind, sodass es zu großen Kontaktflächen zwischen dem Blut und dem Filtermaterial kommt, was zu den oben beschriebenen Aktivierungen der Blutzellen führt.

Alternativ werden Membranen mit einer Porengröße von 5 - 15 *µ*m zur Leukozytenfilterung eingesetzt. Solche Membranen sind beispielsweise im US-Patent 5,820,755 beschrieben. Diese Membranen bestehen aus Nitrozellulose. Im JP-Patent 3-47131 werden für die Leukozytenfilterung Membranen mit einer Dicke von 0,3 - 0,9 mm aus Polyurethan und Membranen aus Polyvinylidenefluoride und Polysulfonen oder Polyester beschrieben.

Der vorliegenden Erfindung liegt die Aufgabe zugrunde, eine Leukozytenfiltervorrichtung für Blut vorzuschlagen, bei der es zu einer geringeren Aktivierung von Blutbestandteilen kommt als bei den bisherigen Leukozytenfiltereinrichtungen.

Die Aufgabe wird mit einer Filtervorrichtung zum Ausfiltrieren von Leukozyten aus Blut, Blutplasma, Blutkomponenten oder Proteinlösungen gelöst, die erfindungsgemäß dadurch gekennzeichnet ist, dass sie ein Vlies und mindestens eine Membran mit einer Dicke von kleiner gleich 150 *µ*m und einer Porengröße von kleiner als 15 *µ*m, aufweist.

Vor dem Vlies kann außerdem eine Membran mit einer Dicke von kleiner gleich 150 *µ*m und einer Porengröße von größer gleich 15 *µ*m angeordnet sein.

Diese vorgeschaltete Membran mit einer relativ großen Porengröße dient der gleichmäßigen Verteilung des Bluts. Das anschließende dünne Vlies hält einen Filterkuchen aus lose zusammenhängenden Leukozyten zurück, während die folgende, feinporige Membran einzelne Leukozyten zurückhält.

Die vorgeschaltete Membran kann aus einem hydrophilen Material gefertigt sein, die schnell von dem Blut, dem Blutplasma oder der Proteinlösung genässt wird. Die Dicke dieser Membran kann vorzugsweise 20 - 150 *µ*m betragen. Durch die geringe Dicke ist der durch diese Membran verursachte Druckverlust des durch die Filtereinrichtung fließenden Bluts nur sehr gering. Die durchschnittliche Porengröße kann 15 - 100 *µ*m, vorzugsweise aber 15 - 40 *µ*m betragen. Die Verteilungsfunktion dieser Membran sorgt dafür, dass der gesamte Filterquerschnitt für das Ausfiltrieren der Leukozyten genutzt wird.

Das nachfolgende Vlies kann eine Porengröße von 15 - 50 *µ*m betragen. Aufgrund dieser großen Porengröße muss das Vlies nicht unbedingt aus einem hydrophilen Material bestehen. Das Blut läuft auch so relativ ungestört durch das Vlies hindurch.

Die dem Vlies nachgeordnete Membran oder Membranen weisen eine Dicke von kleiner als 150 *µ*m, vorzugsweise von 50 - 130 *µ*m auf. Die mittlere Porengröße kann vorzugsweise 4 - 14 *µ*m betragen, sodass Leukozyten die Poren nicht mehr passieren können. Die kleineren Thrombozyten und die Erythrozyten werden jedoch hindurchgelassen. Die Erythrozyten weisen zwar eine ähnliche Größe wie die Leukozyten auf, doch sind sie flexibler und leichter zu deformieren, sodass sie eher im Gegensatz zu den Leukozyten durch die kleinen Poren hindurchgelangen können. Auch die dem Vlies nachgeordnete Membran besteht vorzugsweise aus einem hydrophilen Material, sodass keine Adsorption von Blutzellen auftritt, das Blut also im Fluss nur wenig gebremst wird.

Leukozyten, die nicht an der Membran anhaften oder von ihr adsorbiert werden, werden durch Diffusion und Konvektion in die Vliesschicht zurücktransportiert, in der sie mit der Zeit aufgrund von schwachen hydrophoben Kräften lose adsorbiert werden. Dieser sich dadurch bildende Filterkuchen ist durchlässig für Blutplättchen und Erythrozyten und stellt somit kein nennenswertes Hindernis für den Blutfluss dar.

Weitere Vorteile ergeben sich, wenn die Membranen aus einem biokompatiblen Material gefertigt sind, um eine Abstoßungsreaktion der Blutbestandteile mit den Membranoberflächen zu vermeiden. Als Materialien kommen beispielsweise Polysulfone, Polyethersulfone oder Verbindungen dieser Stoffe mit Polyvinylpyrrolidonen oder ihren Kopolymeren in Frage. Das Vlies kann aus Polyester oder aus Polyolefinen gefertigt sein.

Für eine besonders feine Filterung kann die Vorrichtung mehrere Schichtfolgen aus Membranen und Vliesen aufweisen. Außerdem kann auch oberhalb der ersten Membran ein sehr grobporiges Vlies mit einer mittleren Porengröße von beispielsweise 30 - 200 *µ*m angeordnet sein, das Mikrogerinsel zurückhält, die die Poren der nachfolgenden Membran verstopfen könnten.

Vergleichsversuche erfindungsgemäßer Vorrichtungen mit Leukozytenfiltern, die lediglich ein dickes Vlies enthalten, oder eine Kombination aus einem dünneren Vlies und einer Membran, haben gezeigt, dass die Leukozytenreduktion bei der erfindungsgemäßen Vorrichtung mit 95 % vergleichbar hoch wie bei den Filtern nach dem Stand der Technik liegt, während sich die im Filtrat noch vorhandene Zahl an Thrombozyten und an Erythrozyten gegenüber Filtervorrichtungen nach dem Stand der Technik erhöhen ließen. So konnten bis zu 81 % der Thrombozyten und bis zu 92 % der Erythrozyten wiedergewonnen werden. Bei Leukozytenfiltern mit Vliesen liegt dagegen die Wiedergewinnungsrate von Thrombozyten bei 77 % und die von Erythrozyten bei 88 %.

Insgesamt kann also gesagt werden, dass Leukozytenfilter gemäß der Erfindung sehr gut für die Bearbeitung von Blutkomponenten für die Transfusion eingesetzt werden können, da sie Leukozyten auf effektive Weise entfernen, während nützliche Blutkomponenten zu einem hohen Grad wiedergewonnen und die Blutbestandteile nur sehr wenig aktiviert werden.

Die Schichtfolge kann zur Leukozytenentfernung von Erythrozytenkonzentraten, die durch Zentrifugation hergestellt werden, eingesetzt werden. Außerdem kann die Schichtfolge zur Leukozytenentfernung bei Spenderblut direkt nach Erythrozyten-, Blutplättchen- oder Blutplasmaeinheiten durch Zentrifugation eingesetzt werden. Die Filtervorrichtung kann auch direkt beim Blutspenden zum Ausfiltern von Leukozyten eingesetzt werden, was Verfahrenszeit und damit Kosten für die Herstellung von Blutkomponenten bedeutet.

Weitere Vorteile ergeben sich, wenn die Membranen und Vliese dampfsterilisierbar sind, um Verunreinigungen des Bluts durch die Filtervorrichtung ausschließen zu können.

Außerdem können die Oberflächen ladungsfrei gestaltet werden, sodass nur eine geringe Blutaktivierung stattfindet. Die Filtration mit der erfindungsgemäßen Vorrichtung kann wie bei den Vorrichtungen nach dem Stand der Technik bei Raumtemperatur durchgeführt werden, ohne dass ein Vorspülen nötig wäre.

## Patentansprüche

1. Filtervorrichtung zum Ausfiltern von Leukozyten aus Blut, Blutplasma, Blutkomponenten oder Proteinlösungen, **dadurch gekennzeichnet, dass** sie ein Vlies und mindestens eine Membran mit einer Dicke von kleiner gleich 150 *µ*m und einer Porengröße von kleiner als 15 *µ*m aufweist.

2. Filtervorichtung nach Anspruch 1, **dadurch gekennzeichnet, dass** sie eine dem Vlies vorgeschaltete Membran mit einer Dicke von kleiner gleich 150 *µ*m und einer Porengröße von größer gleich 15 *µ*m aufweist.

3. Filtervorrichtung nach Anspruch 2, **dadurch gekennzeichnet, dass** die Porengröße der dem Vlies vorgeschalteten Membran zwischen 15 - 40 *µ*m beträgt.

4. Filtervorrichtung nach einem der Ansprüche 1 bis 3, **dadurch gekennzeichnet, dass** die Porengröße der einen und/oder mehreren Membranen 4 - 14 *µ*m beträgt.

5. Filtervorrichtung nach einem der Ansprüche 1 bis 4, **dadurch gekennzeichnet, dass** die Membranen aus einem hydrophilen Material gefertigt sind.

6. Filtervorrichtung nach einem der Ansprüche 1 bis 5, **dadurch gekennzeichnet, dass** die Membranen aus einem biokompatiblen Material gefertigt sind.

7. Filtervorrichtung nach einem der Ansprüche 1 bis 6, **dadurch gekennzeichnet, dass** die Membranen aus Polysulfonen, Polyethersulfonen oder Verbindungen dieser Stoffe mit Polyvinylpyrrolidonen oder Ihren Kopolymeren gefertigt sind.

8. Filtervorrichtung nach einem der Ansprüche 1 bis 7, **dadurch gekennzeichnet, dass** das Vlies aus Polyester oder aus Polyolefinen gefertigt ist.

9. Filtervorrichtung nach einem der Ansprüche 1 bis 8, **dadurch gekennzeichnet, dass** sie mehrere Schichtfolgen aus Membranen und Vliesen aufweist.

10. Filtervorrichtung nach einem der Ansprüche 1 bis 9, **dadurch gekennzeichnet, dass** oberhalb der ersten Membran ein Vlies zum Ausfiltern von Gerinseln angeordnet ist.

11. Filtervorrichtung nach einem der Ansprüche 1 bis 10, **dadurch gekennzeichnet, dass** die Membranen und das oder die Vliese dampfsterilisierbar sind.
